# EUROPEAN PATENT SPECIFICATION

(11) **EP 1 482 900 B1**
(45) Date of publication and mention of the grant of the patent: **16.11.2005**
(21) Application number: 03743807.4
(22) Date of filing: 18.02.2003
(51) Int. Cl.: A61K 7/06, A61K 7/09

(54) **TRIBLOCK COPOLYMERS FOR COSMETIC OR PERSONAL CARE COMPOSITIONS**
TRIBLOCK-COPOLYMERE FÜR KOSMETIK- ODER PFLEGEZUSAMMENSETZUNGEN
COPOLYMERES TRIBLOCS POUR COMPOSITIONS DE SOINS PERSONNELS OU COSMETIQUES

(30) Priority: 12.03.2002 EP 02251712
(43) Date of publication of application: 08.12.2004
(73) Proprietor: UNILEVER N.V., 3013 AL Rotterdam (NL); UNILEVER PLC, London EC4P 4BQ (GB)
(72) Inventor: ADAMS, Gerald, Unilever R & D Port Sunlight, Bebington, Wirral, Merseyside CH63 3JW (GB); EASON, Michael D, Unilever R & D Port Sunlight, Merseyside CH63 3JW (GB); KHOSHDEL, Ezat, Unilever R & D Port Sunlight, Bebington, Wirral, Merseyside CH63 3JW (GB); ROGERS, Susanne, H, Unilever R & D Port Sunlight, Bebington, Wirral, Merseyside CH63 3JW (GB)
(74) Representative: Tansley, Sally Elizabeth
(86) International application number: PCT/EP2003/001581
(87) International publication number: WO 2003/075867

(56) References cited:
- WO-A-00/71606
- WO-A-95/01384
- US-A- 5 271 930
- US-A- 5 919 830

## Description

### Field of the Invention

The present invention relates to polymers, to cosmetic or personal care compositions comprising the polymers, and to their use in the treatment of hair.

### Background and Prior Art

Cosmetic and personal care compositions such as hair styling sprays, mousses, gels, shampoos and conditioners, frequently contain resins, gums and adhesive polymers to provide a variety of benefits, for example, film-forming ability, thickening, sensory properties and hair shaping and setting.

It is known that compositions containing certain known hair styling polymers can perform poorly under conditions of high humidity. Thus, hair styled with these compositions can lose its shape and any other benefits conferred by the compositions when the ambient humidity increases or the hair and/or the scalp becomes damp.

Polymers for use in cosmetic and personal care compositions, such as for styling hair, are usually linear or graft homo-or co-polymers which contain various monomers in an alternating, random manner.

Graft copolymers are known for use as film-forming polymers in hair care and other personal care compositions. These graft copolymers typically comprise a polymeric backbone and one or more macromonomers grafted to the backbone, in which the physical and chemical attributes such as glass transition temperature and water solubility can be selected independently for the polymeric backbone and macromonomer grafts in order to provide the desired overall polymer properties.

For example, WO95/01383 and WO95/01384 describe the use of water or alcohol soluble or dispersible graft copolymers in hair and skin care compositions, in which the copolymer has a backbone and two or more polymeric side chains, and is formed by copolymerisation of randomly repeating monomer units A and B. Monomer A is selected to have a hydrophobic character and macromonomer B comprises a long hydrophilic part. EP-A-0412704, EP-A-0408313 and EP-A-0412707 have suggested the use of silicone grafted acrylate copolymers in hair care applications. US 4,988,506 describes the use of non-pressure sensitive polysiloxane-grafted copolymers in hair care compositions. US 5,986,015 describes graft copolymers and hair care compositions containing them.

Block copolymers have an advantage over graft copolymers in that the polymer architecture can be controlled more readily. This is particularly important when designing polymers with segments of distinct physical and chemical properties for particular applications. Graft copolymers typically have many side chains and are not characterised as block copolymers.

WO 98/53794 discusses (AB)ₙ block copolymers, where A is a silicone block and B is a vinylic block. US 5,271,930 (Fintex Inc) describes benzoate esters of polyalkoxylated block copolymers, such as PLURONICS™, for skin and hair care compositions. US 5,472,686 and US 5,660,819 (both Nippon Unicar) describe non-hydrolysing block copolymers comprising a linear polysiloxane-polyoxyalkylene block as a repeating unit in cosmetic formulations. US 5,965,115 and US 5,972,356 (both Procter & Gamble) describe the use of silicone-polyoxyalkylene copolymer surfactants for improving stability of a polyorganosilicone emulsion in personal care compositions.

ABA triblock copolymers where the A blocks are hard and the B block is soft, are generally referred to as "thermoplastic elastomers". Such polymers are multiphase compositions in which the phases are intimately dispersed. A well known material of this type is, for example, poly(styrene-b-butadiene-b-styrene) commonly known as Kraton™ and available from Shell Chemicals Company. However, these materials are only soluble in common organic solvents such as toluene and they are not soluble in cosmetically acceptable solvents such as water and ethanol.

US 5,980,878 discloses thermoplastic elastomers for use in hair and skin care compositions which have a backbone polymer and a plurality of polymeric side chains bound along the length of the backbone polymer. The polymers described, which have a backbone and a plurality of pendant polymer chains, are effectively graft copolymers.

International applications numbers PCT/EP00/04225 and PCT/EP00/04429 describe polysiloxane block copolymers which are built up from units of the formula [A] and [B], in which A is a polymeric block built up from radically polymerisable monomer and B is a polysiloxane block. The block copolymers may be used in cosmetic and personal care compositions.

Commonly, all of the above styling resins either have a carbon backbone composed of various hydrophilic and hydrophobic vinylic monomers or comprise a hydrophobic silicone block. The styling resins can be nonionic but most modern resins carry a charge, usually anionic in character. The hydrophilic monomer is employed to render the polymer water soluble and the hydrophobic monomer or block is selected to enhance humidity resistance of the polymer. Traditionally, the anionically charged resins are usually neutralised with alkalising agents such as sodium or potassium hydroxide as well as certain functional amines such as aminomethyl propanol (AMP) to tailor their solubility and film forming properties.

The hydrophobic/hydrophilic character of modern styling resins is carefully balanced to produce materials that are soluble in hydroalcoholic solvents, typically 80% VOC (volatile organics content). To improve the performance of modern styling products even further, optionally nonvolatile plasticisers such as propylene glycol, dipropylene glycol, acetyl tri-n-butyl citrate and acetyl tri-2-ethoxyhexyl citrate (Citroflex RTM) have been employed.

Accordingly, it is highly desirable to design a styling resin with a balance of hydrophilic and hydrophobic segments to achieve a complex combination of performance properties, e.g. style retention at high humidity, tackiness, flaking, washability from hair and other subjective performance attributes. A typical example of a random copolymer having a good hydrophilic/hydrophobic balance is disclosed in US 3,914,403. This patent discloses a film forming resin obtained by random copolymerisation of vinyl pyrrolidone, vinyl acetate and a cationic monomer. The vinyl pyrrolidone and the cationic monomer form the hydrophilic segment of the resin, while vinyl acetate provides the hydrophobic segment. By varying the ratio of these monomers, water soluble or water insoluble polymers are obtained. Similarly, US 3,925,542 and US 5,196495 disclose further examples of random copolymers with balanced hydrophilic/hydrophobic character. Uses for these random copolymers include aerosols, laquers, non-aerosol hair sprays, hair setting lotions and creams. However, in spite of their hydrophobic component, the prior art random copolymers generally perform poorly under conditions of high humidity.

Surprisingly, we have found that ABA block copolymers where the mid-block, (B), is a poly(alkylene glycol) not only show good solubility in water/alcohol solvent systems but also perform very well under high humidity conditions. This effective performance at high humidity is totally unexpected given the hydrophilic nature of the poly(alkylene glycol) block.

Therefore, it is an object of the present invention to provide polymers, which may be used in cosmetic and personal care compositions, such as hair styling compositions, which exhibit advantages over the compositions of the prior art. In particular, the invention aims to provide a novel class of polymers for use in hair styling compositions. It is a further aim of the present invention to provide cosmetic and personal care compositions (e.g., hair styling compositions) which have relatively good solubility properties (eg, in water, an alcohol such as ethanol or another cosmetically acceptable solvent) and yet perform surprisingly well under conditions of high humidity.

### Summary of the Invention

In a first aspect, the present invention provides a hair treatment composition comprising a polymer comprising an ABA block copolymer, wherein the A groups are polyacrylate blocks or polymethacrylate blocks and the B group is a polyethylene glycol) together with a cosmetically acceptable diluent or carrier.

In another aspect, the present invention provides a cosmetic method of treating hair, which comprises applying to the hair a composition according to the invention.

A further aspect of the invention is the use of a composition of the invention for the cosmetic treatment of hair.

### Detailed Description of the Invention

The present invention is based on the finding of a novel class of polymers for use in cosmetic and personal care compositions, for example hair styling compositions. The polymers are or comprise ABA block copolymers in which the B block is a poly(alkylene glycol) polymer chain. The term poly(alkylene glycol), as used herein, includes polymeric groups obtainable by the polycondensation of alkylene glycols, as well as polymeric groups obtainable by the polymerisation of an alkylene oxide or a cyclic ether (such as tetrahydrofuran). Therefore, the B block may have zero, one, two or more hydroxyl (OH) groups at the end of its chain or its chains. The A blocks are of a polymer that is derived from an ethylenically unsaturated polymerisable monomer. The A blocks may be the same or different but are preferably the same.

The polymers of the invention may simply be the ABA block copolymers or they may comprise the ABA block copolymers, with further modification such as, for example, by grafting onto the ABA block copolymer or by further reaction of the ABA block copolymer.

The polymers of the invention are preferably soluble in a solvent selected from water, ethanol and mixtures thereof. Typically, the polymers are soluble in these solvents at a level of at least 1% by weight, more preferably at least 5% by weight, most preferably at least 10 % by weight, at 25°C.

### ABA Block Copolymers

The ABA block copolymers of the invention are obtainable by bonding each end of a poly(alkylene glycol) B block to an A block. The B block may be linear, branched or hyperbranched and is preferably linear. If the B block is branched or hyperbranched, the block copolymer may comprise two or more A blocks, i.e., it may have the general formula B(A)ₙ, where n is an integer of two or more, preferably from 2 to 6. Polymers of this type are, therefore, encompassed by the term ABA block copolymers as used herein.

Each A block may be bonded to the B block at the same time or each A block may be bonded to the B block in separate reaction steps. A blocks may be bonded directly to B blocks, where the chemistry of the A and B blocks permits this. Alternatively, the A blocks may be bonded to the B blocks via a suitable linker group L, so that the ABA block copolymer is of the formula A-L-B-L-A or, where the B block is branched or hyper branched, B(-L-A)ₙ. Polymers of formula A-L-B-L-A and B(-L-A)ₙ fall within the meaning of the term ABA block copolymer, as used herein. Typically, L is a divalent group having a molecular weight of from 14 to 200 Daltons which links the A and B blocks, preferably via O-C, N-C or S-C bonds to the B block. Preferably L is selected from:

-R-C(O)-O- ;

-R-O-C(O)-O- ;

-R-C(O)-N(R')- ;

-R-O-C(O)-N(R)- ;

or

-R-N(R')-C(O)-N(R")- ;

in which R is a divalent, optionally substituted, linear or branched C₁-C₁₈ hydrocarbon radical (such as C₁-C₁₂ alkylene), and
R' and R'' are independently selected from monovalent, optionally substituted, linear or branched C₁₋₁₈ hydrocarbon radicals e.g., C₁-C₁₈ alkyl, such as methyl.

Preferably, the divalent linker group is a carbonylalkylene group containing from 2 to 7 carbon atoms which forms an ester linkage to the B block, such as, for example, a group of formula -C (O) -C (CH₃)₂-.

Examples of monovalent, unsubstituted radicals are alkyl radicals, such as the methyl, ethyl, n-propyl, iso-propyl, n-butyl, iso-butyl, tert-butyl, n-pentyl, iso-pentyl, neopentyl and tert-pentyl radical; alkoxy radicals, such as the methoxy, ethoxy, n-propoxy, iso-propoxy, n-butoxy, iso-butoxy, tert-butoxy, n-pentoxy, iso-pentoxy, neo-pentoxy and tert-pentoxy radicals; hexyl radicals, such as the n-hexyl radical; alkenyl radicals, such as the vinyl, allyl, 5-hexenyl, 4-vinylcyclohexyl and the 3-norbornenyl radical; cycloalkyl radicals, such as cyclopentyl, cyclohexyl, 4-ethylcyclohexyl and cycloheptyl radical; norbornyl radicals and methylcyclohexyl radicals; aryl radicals, such as the phenyl, biphenylyl, napthyl, anthryl and phenanthryl radical; alkaryl radicals, such as o-, m- and p-tolyl radical, xylyl radicals and ethylphenyl radical; and aralkyl radicals, such as the benzyl, styryl, and phenylethyl radicals.

Examples of monovalent, substituted radicals are halogenated hydrocarbon radicals, such as the chloromethyl, 3-chloropropyl, 3-bromopropyl, 3,3,3-trifluoropropyl and 5,5,5,4,4,3,3-heptafluoropentyl radical and the chlorophenyl, dichlorophenyl and trifluorotolyl radical; mercaptoalkyl radicals, such as the 2-mercaptoethyl and 3-mercaptopropyl radical; cyanoalkyl radicals, such as the 2-cyanoethyl and 3-cyanopropyl radical; aminoalkyl radicals, such as the 3-aminopropyl, N-(2-aminoethyl)-3-aminopropyl and N-(2-aminoethyl)-3-amino-(2-methyl)propyl radical; aminoaryl radicals, such as the aminophenyl radical; acyloxyalkyl radicals, such as the 3-acryloxypropyl and 3-methacryloxypropyl radical; and hydroxyalkyl radicals, such as the hydroxypropyl radical.

Preferred monovalent radicals are independently selected from unsubstituted or substituted C₁ to C₆ alkyl radicals or optionally substituted phenyl radicals, in particular methyl, ethyl, propyl or phenyl radicals.

Examples of divalent hydrocarbon radicals are linear or branched saturated alkylene radicals, such as the methylene and ethylene radical, as well as propylene, butylene, pentylene, hexylene, cyclohexylene and octadecylene radicals; alkoxyalkylene radicals such as the methoxyethylene and ethoxyethylene radical; unsaturated alkylene or arylene radicals, such as the hexenylene radical and phenylene radicals; alkarylene radicals such as the methylphenylene and ethylphenylene radical, and alkoxyarylene radicals such as the methoxyphenylene and ethoxyphenylene radical. The divalent hydrocarbon radical R can be interrupted by divalent radicals, bonded to carbon atoms on both sides, such as -O-, -C(O)O-, -O(O)C-, -CONR⁶-, -NR⁶C (O) - and -C (O) -, where R⁶ is hydrogen or a monovalent, optionally substituted, linear or branched C₁₋₁₈ hydrocarbon radical as described above.

The polymers of the invention may comprise the ABA block copolymers having further polymer chains grafted onto the block copolymer. Suitable polymer chains for grafting onto the block copolymers include, for example, silicones, and polymers derived from monomers such as acrylate and methacrylate esters (eg, esters of acrylic or methacrylic acid with C₁-C₈ straight or branched chain alcohols), styrene (optionally substituted with one or more C₁-C₁₂ straight or branched chain alkyl groups) and mixtures thereof. The polymer chains which may be grafted onto the block copolymers may be hydrophobic or hydrophilic and hydrophobic, hydrophilic or mixtures of hydrophobic and hydrophilic chains may be grafted onto the block copolymers.

Suitable hydrophobic and hydrophilic macromers for the grafts are described in WO 95/06078.

### The A Blocks

The A blocks in the copolymers of the invention are polyacrylate blocks or polymethacrylate blocks.

By "polymerisable" is meant monomers that can be polymerised by reaction between the monomers to form an extended polymer chain which is typically linear.

By "ethylenically unsaturated" is meant monomers that contain at least one polymerisable carbon-carbon double bond (which can be mono-, di-, tri- or tetra-substituted). Either a single monomer or a combination of two or more monomers can be utilised. In either case, the monomers are selected to meet the physical and chemical requirements of the final ABA block copolymer.

The A blocks, which may be the same or different in each ABA block copolymer (but which are preferably the same) preferably have a molecular weight in the range of from 100 to 1,000,000 Daltons, more preferably from 1,000 to 500,000 Daltons.

Suitable ethylenically unsaturated monomers include those having the following general formula:

H(R¹¹) C = C (R¹²) (C (O) G)

in which R¹¹ and R¹² are independently selected from hydrogen, C₁-C₁₀ straight or branched chain alkyl, methoxy, ethoxy, 2-hydroxyethoxy, 2-methoxyethyl and 2-ethoxyethyl groups;
G is selected from hydroxyl, -O(M)_{2/v} , -OR¹³, -NH₂ , -NHR¹³ and - N(R¹³) (R¹⁴);
where M is a counter-ion of valency v selected from metal ions such as alkali metal ions and alkaline earth metal ions, ammonium ions and substituted ammonium ions such as mono-, di-, tri- and tetraalkylammonium ions, and each R¹³ and R¹⁴ is independently selected from hydrogen, C₁-C₄₀ straight or branched chain alkyl, optionally substituted with one or more groups selected from hydroxy, amino, C₁-C₃ alkoxy, C₁-C₃ alkylamino and di (C₁-C₃ alkyl) amino, for example N,N-dimethylaminoethyl, 2-hydroxyethyl, 2-methoxyethyl, and 2-ethoxyethyl. Representative non-limiting examples of monomers useful herein include protected or non-protected acrylic acid and methacrylic acid and salts, esters and amides thereof.

The salts can be derived from any of the common nontoxic metal, ammonium, or substituted ammonium counter ions. The esters can be derived from C₁₋₄₀ straight chain, C₃₋₄₀ branched chain, or C₃₋₄₀ carbocyclic alcohols, from polyhydric alcohols having from about 2 to about 8 carbon atoms and from about 2 to about 8 hydroxyl groups (non-limiting examples of which include ethylene glycol, propylene glycol, butylene glycol, hexylene glycol, glycerol, and 1,2,6-hexanetriol); from amino alcohols (non-limiting examples of which include aminoethanol, dimethylaminoethanol and diethylaminoethanol and their quaternised derivatives); or from alcohol ethers (non-limiting examples of which include methoxyethanol and ethoxyethanol).

The amides can be unsubstituted, N-alkyl or N-alkylamino mono-substituted, or N,N-dialkyl, or N,N-dialkylamino disubstituted, wherein the alkyl or alkylamino groups can be derived from C₁₋₄₀ straight chain, C₃₋₄₀ branched chain, or C₃-₄₀ carbocyclic moieties. In addition, the alkylamino groups can be quaternised.

Also useful as monomers are protected and unprotected acrylic or/and methacrylic acids, salts, esters and amides thereof, wherein the substituents are on the two and three carbon position of the acrylic and/or methacrylic acids, and are independently selected from C₁₋₄ alkyl, hydroxyl, halide (-Cl,-Br,-F,-I), -CN, and -CO₂H, for example methacrylic acid, ethacrylic acid, alpha-chloroacrylic acid and 3-cyano acrylic acid. The salts, esters, and amides of these substituted acrylic and methacrylic acids can be defined as described above for the acrylic/methacrylic acid salts, esters and amides.

Other useful monomers include: vinyl and allyl esters of C₁₋₄₀ straight chain, C₃₋₄₀ branched chain, or C₃₋₄₀ carbocyclic carboxylic acids; vinyl and allyl halides (e.g. vinyl chloride, allyl chloride); pyridines substituted with one or more vinyl or allyl groups (e.g. vinyl pyridine, allyl pyridine); vinylidene chloride; and hydrocarbons having at least one unsaturated carbon-carbon double bond (e.g. styrene, alpha-methylstyrene, t-butylstyrene, butadiene, isoprene, cyclohexadiene, ethylene, propylene, 1-butene, 2-butene, isobutylene, p-methylstyrene); and mixtures thereof.

Preferred monomers useful herein include those selected from protected and unprotected acrylic acid, methacrylic acid, ethacrylic acid, methyl acrylate, ethyl acrylate, n-butyl acrylate, iso-butyl acrylate, t-butyl acrylate, 2-ethylhexyl acrylate, decyl acrylate, octyl acrylate, methyl methacrylate, ethyl methacrylate, n-butyl methacrylate, iso-butyl methacrylate, t-butyl methacrylate, 2-ethylhexyl methacrylate, decyl methacrylate, methyl ethacrylate, ethyl ethacrylate, n-butyl ethacrylate, iso-butyl ethacrylate, t-butyl ethacrylate, 2-ethylhexyl ethacrylate, decyl ethacrylate, 2,3-dihydroxypropyl acrylate, 2,3-dihydroxypropyl methacrylate, 2-hydroxyethyl acrylate, 2-hydroxypropyl acrylate, hydroxypropyl methacrylate, glyceryl monoacrylate, glyceryl monoethacrylate, glycidyl methacrylate, glycidyl acrylate, acrylamide, methacrylamide, ethacrylamide, N-methyl acrylamide, N,N-dimethyl acrylamide, N,N-dimethyl methacrylamide, N-ethyl acrylamide, N-isopropyl acrylamide, N-butyl acrylamide, N-t-butyl acrylamide, N,N-di-n-butyl acrylamide, N,N-diethylacrylamide, N-octyl acrylamide, N-octadecyl acrylamide, N,N-diethylacrylamide, N-phenyl acrylamide, N-methyl methacrylamide, N-ethyl methacrylamide, N-dodecyl methacrylamide, N,N-dimethylaminoethyl acrylamide, quaternised N,N-dimethylaminoethyl acrylamide, N,N-dimethylaminoethyl methacrylamide, quaternised N,N-dimethylaminoethyl methacrylamide, N,N-dimethylaminoethyl acrylate, N,N-dimethylaminoethyl methacrylate, quaternised N,N-dimethylaminoethyl acrylate, quaternised N,N-dimethylaminoethyl methacrylate, 2-hydroxyethyl acrylate, 2-hydroxyethyl methacrylate, 2-hydroxyethyl ethacrylate, glyceryl acrylate, 2-methoxyethyl acrylate, 2-methoxyethyl methacrylate, 2-methoxyethyl ethacrylate, 2-ethoxyethyl acrylate, 2-ethoxyethyl methacrylate, 2-ethoxyethyl ethacrylate, maleic acid, maleic anhydride and its half esters, fumaric acid, itaconic acid, itaconic anhydride and its half esters, crotonic acid, angelic acid, diallyldimethyl ammonium chloride, vinyl pyrrolidone, vinyl imidazole, methyl vinyl ether, methyl vinyl ketone, maleimide, vinyl pyridine, vinyl furan, styrene sulphonate, allyl alcohol, allyl citrate, allyl tartrate, vinyl acetate, vinyl alcohol, vinyl caprolactam and mixtures thereof.

It is particularly preferred that the A groups are polyacrylate blocks or polymethacrylate blocks.

Therefore, more preferred monomers are those selected from methyl acrylate, methyl methacrylate, methyl ethacrylate, ethyl acrylate, ethyl methacrylate, ethyl ethacrylate, n-butyl acrylate, n-butyl methacrylate, n-butyl ethacrylate, 2-ethylhexyl acrylate, 2-ethylhexyl methacrylate, 2-ethylhexyl ethacrylate, N-octyl acrylamide, 2-methoxyethyl acrylate, 2-hydroxyethyl acrylate, N,N-dimethylaminoethyl acrylate, N,N-dimethylaminoethyl methacrylate, acrylic acid, methacrylic acid, 2-hydroxyethyl acrylate, 2-hydroxyethyl methacrylate and mixtures thereof.

Most preferred monomers are those selected from N,N-dimethylaminoethyl acrylate, N,N-dimethylaminoethyl methacrylate, 2-ethylhexyl acrylate, hydroxyethyl methacrylate, N-octyl acrylamide, 2-hydroxyethyl acrylate, 2-hydroxyethyl methacrylate and mixtures thereof.

Even more preferably, the A groups are poly(aminoalkyl methacrylate) blocks, such as, for example, poly(2-(dimethylamino)ethyl methacrylate) blocks.

### The B Block

The B block in the ABA block copolymers of the invention is a poly(ethylene glycol).

The poly(alkylene glycol) block of the B block may be obtainable from a single alkylene glycol, alkylene oxide or cyclic ether or a mixture of two or more different alkylene glycols, alkylene oxides or cyclic ethers. The poly(alkylene glycol) block, when not bound in the ABA block copolymers of the invention, may have zero, one, two or a plurality of hydroxyl groups at the ends of its chain or, when the B block is not linear, its chains. Although the poly(alkylene glycol) block may contain other than alkyleneoxy groups, it preferably comprises at least 75%, more preferably at least 90%, most preferably at least 99% by weight of the B block of alkyleneoxy groups.

Suitable alkyleneoxy groups in the B block include groups of formula -R"'-O-, wherein R"' is a straight chain or branched aliphatic, saturated or unsaturated, C₂ to C₁₂ alkylene group or an alicyclic, saturated or unsaturated C₃ to C₁₂ cycloalkylene group. Preferably, R"' is a straight chain or branched saturated aliphatic C₂ to C₆ group, more preferably a C₂ to C₄ group. For example, the B group may comprise repeating units selected from ethyleneoxy, propyleneoxy and mixtures thereof. Most preferably, the B group is a poly(ethylene glycol).

The B group may be linear, branched or hyperbranched. Where the B group is branched or hyperbranched, it may comprise two or more A blocks, with each A block bonded to an end of a chain in the B block.

The B block may be derived from a single monomer or may comprise residues derived from two or more different monomers. When the B block is derived from two or more monomers, the B block may be a block copolymer or a random copolymer. For example, the B block may be a block copolymer comprising C-D-C blocks e.g., the B block may be a poly(ethylene glycol) - poly(propylene glycol) - poly(ethylene glycol) block copolymer. When the B block is a block copolymer of formula C-D-C, the ABA block copolymer of the invention has the formula A-C-D-C-A.

Particularly preferred poly(alkylene glycol)s corresponding to the above general formula comprise from 5 to 1,000,000, preferably from 5 to 500 repeating alkyleneoxy units.

### Process for producing ABA block copolymers

The polymers of the present invention may be formed by a number of different routes. However, the polymers are preferably formed according to the process of the invention. In a preferred embodiment, the process of the invention involves a reaction which comprises two key reaction steps:

### First reaction step

The first reaction step involves forming a poly(alkylene glycol) macroinitiator by grafting a radical initiator onto a poly(alkylene glycol) via a nucleophilic displacement reaction between groups on the poly(alkylene glycol) and on the radical initiator, respectively.

Typically the poly(alkylene glycol) macroinitiator is formed by a nucleophilic displacement reaction between:
(i) a poly(alkylene glycol) which is end-capped with at least one group capable of nucleophilic attack via its O, N or S atom,
   and
(ii) a radical initiator comprising: at least one -C(O)X group, in which X is a leaving group capable of substitution by the nucleophilic O ,N or S atom of the end-capped poly(alkylene glycol) (i); and at least one organic halide group capable of generating a radical, preferably in the presence of a transition metal catalyst. The term halide means fluoride, chloride, bromide or iodide.

The poly(alkylene glycol) (i) may be linear, branched or hyperbranched, provided it is end-capped with at least one group as described above. By "end-capped" is meant that the capping group is at or near a terminal position of the poly(alkylene glycol). The poly(alkylene glycol) may be end-capped with suitable groups (e.g., hydroxyl) as a result of its synthesis or an additional reaction step may be required to end-cap the polymer chain.

The radical initiator (ii) comprises at least one -C(O)X group, in which X is a leaving group capable of substitution by the nucleophilic O, N or S atom of the polyethyleneglycol (i), and at least one organic halide group capable of generating a radical in the presence of a transition metal catalyst.

Examples of preferred radical initiators have the formula:

R⁷-C(O)X

where R⁷ is the organic halide group and X is the leaving group. Preferably, X is a halogen atom (i.e., F, Cl, Br or I). By "organic halide group" is meant any linear, branched or cyclic (aromatic or otherwise) carbon structure, whether substituted or unsubstituted, which also contains a halogen atom (i.e., F, Cl, Br or I).

Preferred radical initiators have the general formula:

C(R⁸) (R⁹)Hal'-(R¹⁰)ᵣ -C(O)Hal

where Hal' and Hal independently denote halogen atoms (as defined above), R⁸ and R⁹ are independently selected from hydrogen or a monovalent, optionally substituted, linear or branched C₁₋₁₈ hydrocarbon radical as described above, r is an integer having a value of 0 or 1, and R¹⁰ is selected from divalent, optionally substituted, linear or branched C₁ - C₁₈ hydrocarbon radicals as described above.

A particularly preferred radical initiator corresponding to the above general formula has:

Hal and Hal' = Br, R⁸ and R⁹= methyl and r = 0.

The first reaction step involves a nucleophilic displacement reaction between (i) and (ii) under conventional reaction conditions. The nucleophilic O, N or S atom of the poly(alkylene glycol) (i) replaces leaving group X of radical initiator (ii), thereby linking (i) and (ii) to generate a poly(alkylene glycol) macroinitiator.

### Second reaction step

The second reaction step involves reacting the organic halide groups of the poly(alkylene glycol) macroinitiator obtained in step (i) with radically polymerisable monomers in the presence of a catalytic or stoichiometric amount of a Cu (I) salt or other transitional metal species to form a poly(alkylene glycol) block copolymer.

In this reaction step, the organic halide groups act as initiators in the presence of the radically polymerisable monomers and the catalyst, resulting in the linking of a block of radically polymerisable monomers onto the poly(alkylene glycol) macroinitiator by atom transfer radical polymerisation. This block of radically polymerisable monomers constitutes the polymeric block (denoted A) of the poly(alkylene glycol) block copolymer of the invention.

The catalyst for the second reaction step is a transition metal salt, preferably a Cu(I) salt such as a Cu(I) halide salt (i.e., wherein the halide is Cl, F, Br or I) and which is preferably complexed to a ligand which is suitable for solubilising the Cu(I) salt in the reaction mixture. WO98/51261 describes preferred ligands for use in solubilising the Cu(I) salt in the reaction mixture (aprotic bidentates such as diphosphates, 2,2' bipyridyl, C₁-C₂₀ alkyl substituted bipyridyl and combinations thereof, most preferably 2,2' bipyridyl complexed to a Cu(I) halide salt, in particular CuCl). WO98/5126 also refers to several journal articles which describe examples of the polymerisation process (atom transfer radical polymerisation) used in the second reaction step of the process of the present invention. Further examples of such descriptions can be found in Polymer Vol 39, No.21, pp 5163-5170 (Nakagawa et al) and *Macromolecules* **1997**, *30*, 2190-2193 (Haddleton et al). Those skilled in the art would understand that a variety of other ligands can also be employed.

The polymerisation process of the second reaction step can be furnished in bulk, solution, emulsion or suspension, as would be understood by those skilled in the art.

Radically polymerisable monomers suitable for use in the second reaction step of the process of the present invention are preferably ethylenically unsaturated monomers.

### Compositions of the invention

Cosmetic or personal care compositions of the present invention are preferably formulated into hair care compositions, especially hairspray compositions, but can also be formulated into a wide variety of product types, including mousses, gels, lotions, tonics, sprays, shampoos, conditioners, rinses, hand and body lotions, facial moisturisers, sunscreens, anti-acne preparations, topical analgesics, mascaras, and the like. Compositions of the invention comprise a cosmetically acceptable diluent or carrier. Preferably, the compositions are for use in styling human hair and, more preferably, they are packaged and labelled as such.

Compositions of the invention preferably contain the polymer in an amount of from 0.01% to 30% (more preferably from 0.1 to 10%) by weight. Compositions of the invention may, optionally, comprise a fragrance or perfume and/or one or more of the optional additional components described hereinafter.

The carriers and additional components required to formulate such products vary with product type and can be routinely chosen by one skilled in the art. The following is a description of some of these carriers and additional components.

### Carriers

Cosmetic or personal care compositions of the present invention can comprise a carrier, or a mixture of such carriers, which are suitable for application to the hair. The carriers are present at from about 0.5% to about 99.5%, preferably from about 5.0% to about 99.5%, more preferably from about 10.0% to about 98.0%, by weight of the composition. As used herein, the phrase "suitable for application to hair" means that the carrier does not damage or negatively affect the aesthetics of hair or cause irritation to the underlying skin.

Carriers suitable for use with hair care compositions of the present invention include, for example, those used in the formulation of hair sprays, mousses, tonics, gels, shampoos, conditioners, and rinses. The choice of appropriate carrier will also depend on the particular polymer to be used, and whether the product formulated is meant to be left on the surface to which it is applied (e.g., hair spray, mousse, tonic, or gel) or rinsed off after use (e.g., shampoo, conditioner, rinse).

The carriers used herein can include a wide range of components conventionally used in hair care compositions. The carriers can contain a solvent to dissolve or disperse the particular polymer being used, with water, the C₁-C₆ alcohols, lower alkyl acetate and mixtures thereof being preferred. The carriers can also contain a wide variety of additional materials such as acetone, hydrocarbons (such as isobutane, hexane, decene), halogenated hydrocarbons (such as Freons) and volatile silicones such as cyclomethicone.

When the hair care composition is a hair spray, tonic, gel, or mousse the preferred solvents include water, ethanol, volatile silicone derivatives, and mixtures thereof. The solvents used in such mixtures may be miscible or immiscible with each other. Mousses and aerosol hair sprays can also utilise any of the conventional propellants to deliver the material as a foam (in the case of a mousse) or as a fine, uniform spray (in the case of an aerosol hair spray). Examples of suitable propellants include materials such as trichlorofluoromethane, dichlorodifluoromethane, difluoroethane, dimethylether, propane, n-butane or isobutane. A tonic or hair spray product having a low viscosity may also utilise an emulsifying agent. Examples of suitable emulsifying agents include nonionic, cationic, anionic surfactants, or mixtures thereof. If such an emulsifying agent is used, it is preferably present at a level of from about 0.01% to about 7.5% by weight based on total weight of the composition. The level of propellant can be adjusted as desired but is generally from about 3% to about 30% by weight based on total weight for mousse compositions and from about 15% to about 50% by weight based on total weight for aerosol hair spray compositions.

Suitable spray containers are well known in the art and include conventional, non-aerosol pump sprays i.e., "atomisers", aerosol containers or cans having a propellant, as described above, and also pump aerosol containers utilising compressed air as the propellant.

Where the hair care compositions are conditioners and rinses, the carrier can include a wide variety of conditioning materials. Where the hair care compositions are shampoos, the carrier can include, for example, surfactants, suspending agents, and thickeners. Hair styling creams or gels also typically contain a structurant or thickener, typically in an amount of from 0.01% to 10% by weight.

The carrier can be in a wide variety of forms. For example, emulsion carriers, including oil-in-water, water-in-oil, water-in-oil-in-water, and oil-in-water-in-silicone emulsions, are useful herein. These emulsions can cover a broad range of viscosities, e.g., from about 100 cps to about 200,000 cps. These emulsions can also be delivered in the form of sprays using either mechanical pump containers or pressurised aerosol containers using conventional propellants. These carriers can also be delivered in the form of a mousse. Other suitable topical carriers include anhydrous liquid solvents such as oils, alcohols, and silicones (e.g., mineral oil, ethanol, isopropanol, dimethicone, cyclomethicone, and the like); aqueous-based single phase liquid solvents (e.g., hydro-alcoholic solvent systems); and thickened versions of these anhydrous and aqueous-based single phase solvents (e.g., where the viscosity of the solvent has been increased to form a solid or semi-solid by the addition of appropriate gums, resins, waxes, polymers, salts, and the like).

### Additional Components

A wide variety of additional components can be employed in compositions according to the present invention. Examples include the following:
- hair styling polymers for hair styling compositions such as hair sprays, gels, and mousses. Hair styling polymers are well known articles of commerce and many such polymers are available commercially which contain moieties which render the polymers cationic, anionic, amphoteric or nonionic in nature. The polymers may be synthetic or naturally derived.

The amount of the polymer may range from 0.5 to 10%, preferably 0.75 to 6% by weight based on total weight of the composition.

Examples of anionic hair styling polymers are:
copolymers of vinyl acetate and crotonic acid;
terpolymers of vinyl acetate, crotonic acid and a vinyl ester of an alpha-branched saturated aliphatic monocarboxylic acid such as vinyl neodecanoate;
copolymers of methyl vinyl ether and maleic anhydride (molar ratio about 1:1) wherein such copolymers are 50% esterified with a saturated alcohol containing from 1 to 4 carbon atoms such as ethanol or butanol;
acrylic copolymers containing acrylic acid or methacrylic acid as the anionic radical-containing moiety with other monomers such as: esters of acrylic or methacrylic acid with one or more saturated alcohols having from 1 to 22 carbon atoms (such as methyl methacrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate); glycols having from 1 to 6 carbon atoms (such as hydroxypropyl methacrylate and hydroxyethyl acrylate); styrene; vinyl caprolactam; vinyl acetate; acrylamide; alkyl acrylamides and methacrylamides having 1 to 8 carbon atoms in the alkyl group (such as methacrylamide, t-butyl acrylamide and n-octyl acrylamide); and other compatible unsaturated monomers.

The polymer may also contain grafted silicone, such as polydimethylsiloxane.

Specific examples of suitable anionic hair styling polymers are:
RESYN® 28-2930 available from National Starch (vinyl acetate/crotonic acid/vinyl neodecanoate copolymer);
ULTRAHOLD® 8 available from BASF (CTFA designation Acrylates/acrylamide copolymer);
the GANTREZ®ES series available from ISP corporation esterified copolymers of methyl vinyl ether and maleic anhydride).

Other suitable anionic hair styling polymers include carboxylated polyurethanes. Carboxylated polyurethane resins are linear, hydroxyl-terminated copolymers having pendant carboxyl groups. They may be ethoxylated and/or propoxylated at least at one terminal end. The carboxyl group can be a carboxylic acid group or an ester group, wherein the alkyl moiety of the ester group contains one to three carbon atoms. The carboxylated polyurethane resin can also be a copolymer of polyvinylpyrrolidone and a polyurethane, having a CTFA designation PVP/polycarbamyl polyglycol ester. Suitable carboxylated polyurethane resins are disclosed in EP-A-0619111 and US Patent No. 5,000,955. Other suitable hydrophilic polyurethanes are disclosed in US Patent Nos. 3,822,238; 4,156,066; 4,156,067; 4,255,550; and 4,743,673.

Amphoteric hair styling polymers which can contain cationic groups derived from monomers such as t-butyl aminoethyl methacrylate as well as carboxyl groups derived from monomers such as acrylic acid or methacrylic acid can also be used in the present invention. One specific example of an amphoteric hair styling polymer is Amphomer® (Octylacrylamide/ acrylates/butylaminoethyl methacrylate copolymer) sold by the National Starch and Chemical Corporation.

Examples of nonionic hair styling polymers are homopolymers of N- vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and are homopolymers of N-vinylpyrrolidone having an average molecular weight of about 1,000,000 sold under the name of PVP K-120.

Other suitable nonionic hair styling polymers are cross-linked silicone resins or gums. Specific examples include rigid silicone polymers such as those described in EP-A-0240350 and cross-linked silicone gums such as those described in WO 96/31188.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;
Polyquaternium-16 (formed from methylvinylimidazolium chloride and vinylpyrrolidone), available from BASF as Luviquat® FC 370, FC 550, FC 905 and HM-552;
Polyquaternium-46 (prepared by the reaction of vinylcaprolactam and vinylpyrrolidone with methylvinylimidazolium methosulphate), available from BASF as Luviquat®Hold.

Examples of suitable naturally-derived polymers include shellac, alginates, gelatins, pectins, cellulose derivatives and chitosan or salts and derivatives thereof. Commercially available examples include Kytamer® (ex Amerchol) and Amaze® (ex National Starch).

Also suitable for use as optional components in the compositions of the invention are the ionic copolymers described in WO 93/03703, the polysiloxane-grafted polymers disclosed in WO 93/23446, the silicone-containing polycarboxylic acid copolymers described in WO 95/00106 or WO 95/32703, the thermoplastic elastomeric copolymers described in WO 95/01383, WO 95/06078, WO 95/06079 and WO 95/01384, the silicone grafted adhesive polymers disclosed in WO 95/04518 or WO 95/05800, the silicone macro-grafted copolymers taught in WO 96/21417, the silicone macromers of WO 96/32918, the adhesive polymers of WO 98/48770 or WO 98/48771 or WO 98/48772 or WO 98/48776, the graft polymers of WO 98/51261 and the grafted copolymers described in WO 98/51755.

With certain of the above-described polymers it may be necessary to neutralise some acidic groups to promote solubility/dispersibility. Examples of suitable neutralising agents include 2-amino-2- methyl-1, 3-propanediol (AMPD); 2-amino-2-ethyl-1,3-propanediol (AEPD); 2-amino-2-methyl-1-propanol (AMP); 2-amino-1-butanol (AB); monoethanolamine (MEA); diethanolamine (DEA); triethanolamine (TEA); monoisopropanolamine (MIPA); diisopropanol-amine (DIPA); triisopropanolamine (TIPA); and dimethyl stearamine (DMS). A long chain amine neutralising agent such as stearamidopropyl dimethylamine or lauramidopropyl dimethylamine may be employed, as is described in US 4,874,604. Also suitable are inorganic neutralisers, examples of which include sodium hydroxide, potassium hydroxide and borax. Mixtures of any of the above neutralising agents may be used. Amounts of the neutralising agents will range from about 0.001 to about 10% by weight of the total composition.
- sunscreening agents such as 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, and mixtures thereof.
- anti-dandruff actives such as zinc pyrithione, piroctone olamine, selenium disulphide, sulphur, coal tar, and the like.
- hair conditioning agents such as hydrocarbons, silicone fluids, and cationic materials. The hydrocarbons can be either straight or branched chain and can contain from about 10 to about 16, preferably from about 12 to about 16 carbon atoms. Examples of suitable hydrocarbons are decane, dodecane, tetradecane, tridecane, and mixtures thereof. Examples of suitable silicone conditioning agents useful herein can include either cyclic or linear polydimethylsiloxanes, phenyl and alkyl phenyl silicones, and silicone copolyols. Cationic conditioning agents useful herein can include quaternary ammonium salts or the salts of fatty amines.
- surfactants for hair shampoo and conditioner compositions. For a shampoo, the level is preferably from about 10% to about 30%, preferably from 12% to about 25%, by weight based on total weight of the composition. For conditioners, the preferred level of surfactant is from about 0.2% to about 3%, by weight based on total weight of the composition. Surfactants useful in compositions of the present invention include anionic, nonionic, cationic, zwitterionic and amphoteric surfactants.
- carboxylic acid polymer thickeners for hair shampoo and conditioner compositions. These crosslinked polymers contain one or more monomers derived from acrylic acid, substituted acrylic acids, and salts and esters of these acrylic acids and the substituted acrylic acids, wherein the crosslinking agent contains two or more carbon-carbon double bonds and derived from a polyhydric alcohol. Examples of carboxylic acid polymer thickeners useful herein are those selected from the group consisting of carbomers, acrylates/C10-C30 alkyl acrylate crosspolymers, and mixtures thereof. Compositions of the present invention can comprise from about 0.025% to about 1%, more preferably from about 0.05% to about 0.75% and most preferably from about 0.10% to about 0.50% of the carboxylic acid polymer thickeners, by weight based on total weight of the composition.
- emulsifiers for emulsifying the various carrier components of the compositions of the invention. Suitable emulsifier types include polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, and mixtures thereof. The emulsifiers can be used individually or as a mixture of two or more and can comprise from about 0.1% to about 10%, more preferably from about 1% to about 7%, and most preferably from about 1% to about 5%, by weight based on total weight of the composition.
- vitamins and derivatives thereof (e.g., ascorbic acid, vitamin E, tocopheryl acetate, retinoic acid, retinol, retinoids, and the like).
- cationic polymers (e.g., cationic guar gum derivatives such as guar hydroxypropyltrimonium chloride and hydroxypropyl guar hydroxypropyltrimonium chloride, available as the Jaguar® series from Rhone-Poulenc).
- preservatives, antioxidants, chelators and sequestrants; and aesthetic components such as fragrances, colourings, hair nutrients and essential oils.

The invention also involves a method of styling hair by applying thereto a styling composition as is hereinabove described.

The following non-limiting Examples further illustrate the preferred embodiments of the invention. All percentages referred to are by weight based on total weight unless otherwise indicated.

### EXAMPLES

### Examples 1 to 3

ABA Triblock copolymers of the following general formula: 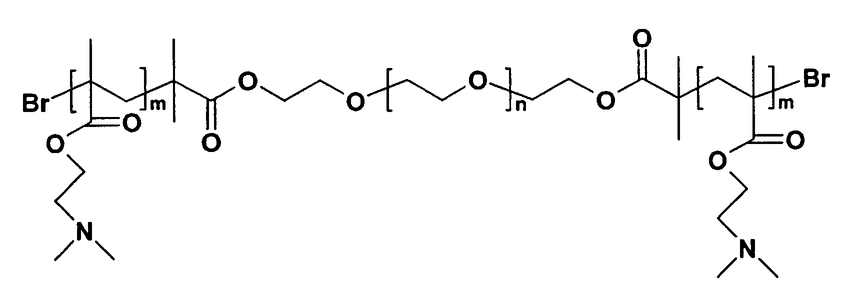 in which m and n are positive integers
were prepared by atom transfer radical polymerisation (ATRP). Commercially available poly(ethylene glycol)s were halide functionalised to give an effective ATRP initiator. Controlled molecular weights were achieved with narrow polydispersities.

### Experimental procedures for the preparation of PDMAEMA - PEG -PDMAEMA - block copolymers

### (A) Preparation of poly(ethylene glycol) (PEG) macroinitiator

Poly(ethylene glycol), MW = 1000, ex Polysciences Inc. (20g, 0.02 moles) was dissolved in anhydrous toluene (100 ml) in a 2-necked round bottom flask fitted with a suba seal and calcium chloride guard tube. 4-(Dimethylamino)pyridine (DMAP) (0.054g, 9.4x10⁻⁴ moles), triethylamine (TEA) (4.45g, 0.044 moles) and a magnetic stirrer bar were added to the PEG solution. 2-Bromoisobutyryl bromide (10.12g, 0.044 moles) was added dropwise via a syringe through the suba seal, which caused the initially clear solution to turn to a milky suspension. After stirring at ambient overnight, the toluene was removed by evaporation using a rotary evaporator and the remaining brown liquid was dissolved in dichloromethane (200ml). This solution was added to a separating funnel and washed successively with saturated sodium bicarbonate solution (100ml), 1M hydrochloric acid (100ml) and brine (100ml). The dichloromethane layer was then dried over magnesium sulfate, filtered and the solvent evaporated. After drying overnight under vacuo the product was obtained as a brown oil (19.25g, 73.1% yield).

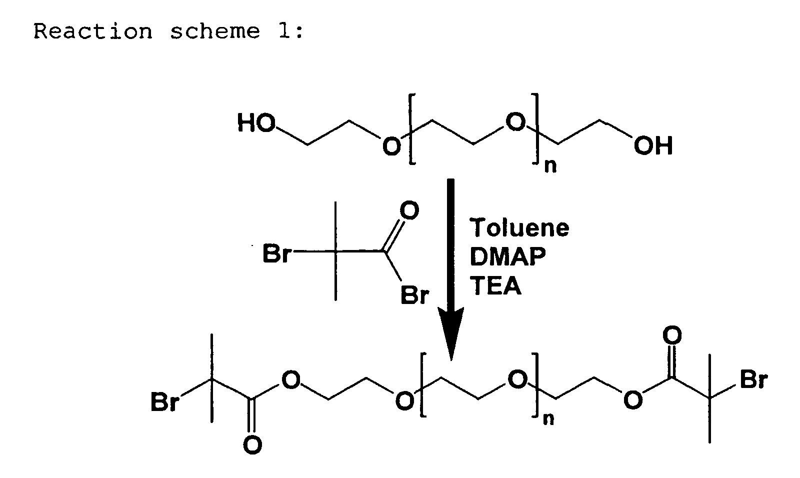

### Characterisation:

**IR :** 1734 cm⁻¹ (s, saturated ester carbonyl)
**NMR (**^{**1**}**H-CDCl**_{**3**}**)** : 1.94 (d, ester CH₃, 12H); 3.64 (s, PEG CH₂, 80H); 3.73 (t, CH₂-O, 4H); 4.33 (t, CH₂-O, 4H).

### (B) Preparation of PDMAEMA- PEG-PDMAEMA block copolymers (PDMAEMA=poly(2-(dimethylamino)ethyl methacrylate))

2-(Dimethylamino)ethyl methacrylate (ex Aldrich) (15.41g, 0.098 moles, the amount required to give a theoretical molecular weight of 5000) and the PEG-macroinitiator (2g, 1.54x10⁻³ moles) were dissolved in demineralised water (20 ml) and added to a 3-necked 50ml round bottom flask fitted with a magnetic stirrer bar, a thermometer, a N₂ inlet and a suba seal fitted with a gas outlet. Dry N₂ gas was bubbled through the solution for 45 minutes. Copper(I)bromide (0.221g, 1.54x10⁻³ moles) and 2,2'-dipyridyl (0.4866, 3.08x10⁻³ moles) were weighed into a glass vial, mixed and added to the reaction mixture by lifting the thermometer from the flask's socket, replacing the thermometer immediately after addition of the solids. The reaction mixture turned to a green colour on mixing the solids into solution, also an increase in viscosity was noted. A reaction exotherm of 28°C was recorded over a time period of 6 minutes. After mixing for 2 hours under a N₂ blanket, the contents of the flask was poured into a beaker and diluted with water (100ml). This solution was then poured through a bed of silica to remove the copper metal, which resulted in the initially dark green solution becoming clear and almost water-white.

This aqueous solution was freeze-dried over the weekend yielding 15g of slightly pink material.

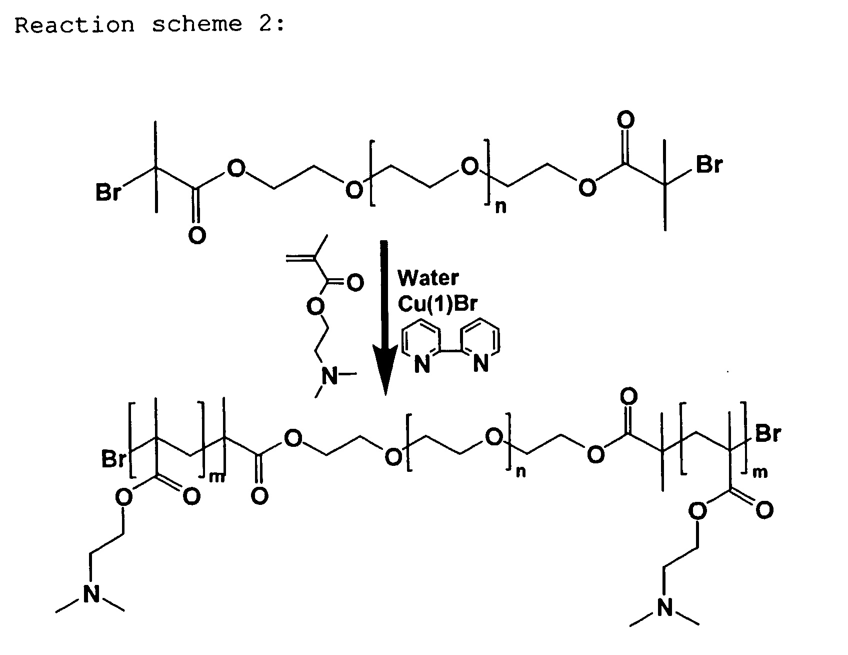

m and n are positive integers

### Characterisation:

**IR :** 1723 cm⁻¹ (s, saturated ester carbonyl)
**NMR (**^{**1**}**H-CDCl**_{**3**}**)** : 2.3 (m, N-CH₃, 6H); 2.6 (s, N-CH₂, 2H) ; 3.65(s, O-CH₂-CH₂-O, 4H); 4.08 (s, CH₂-O, 2H).
**GPC (eluent = THF; PMMA standards)** : Mn = 9000; Mw = 11900; Pdi = 1.35

Similarly, the copolymers of Examples 2 and 3 with poly(ethylene glycol)s of different sizes were prepared.
Example 1 - Poly(DMAEMA)-(PEG)-(DMAEMA) [1k PEG; 26933/88]
Example 2 - Poly(DMAEMA)-(PEG)-(DMAEMA) [5.5k PEG; 26933/92]
Example 3 - Poly(DMAEMA)-(PEG)-(DMAEMA) [7.5k PEG; 26933/96]

### Viscosity bond strength and measurements

### Materials

The materials of Examples 1 to 3 were successfully formulated into a 55% VOC pumpspray solution containing, by weight based on total weight of the composition, 3% solids, 55% ethanol and 42% water.

### Viscosity measurements

The viscosity of the 3% solids formulations prepared using the copolymers of Examples 1 to 3 was measured using the Brookfield viscometer and low viscosity spindle (SOO). 20 ml of sample was used and the speed set at 60 rpm.

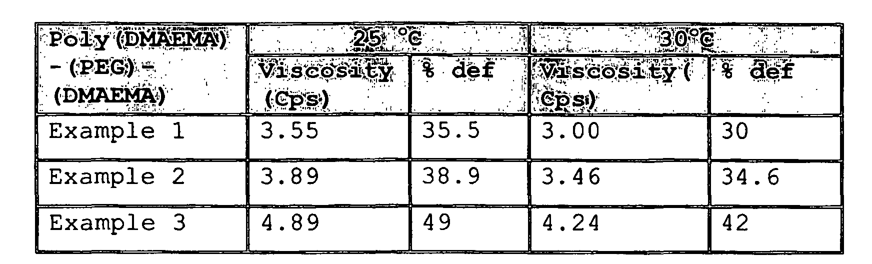

The viscosity measurements indicate that no problems should be experienced with spraying these solutions.

### Bond strength Analysis

1µl of the 3% solids formulations prepared using the copolymers of Examples 1 to 3 was pipetted onto a hair junction. The following bond strengths were obtained.

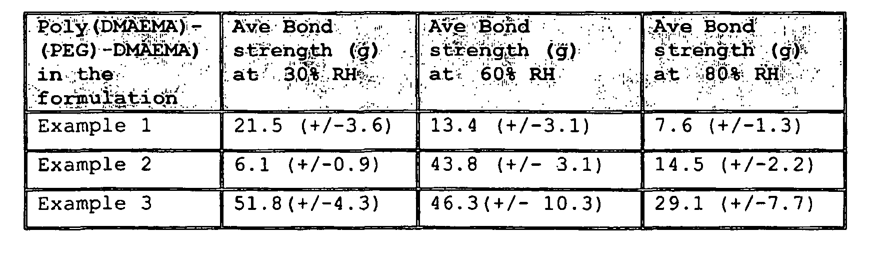

All failure mechanisms for 30 and 60% RH were mainly adhesive with a small amount of cohesion (a small amount of resin left on fixed fibre). At 80% RH all bond failed cohesively and was ductile.

### Examples 4 to 9

The following are examples of compositions according to the invention.

The materials used in the examples include the following:

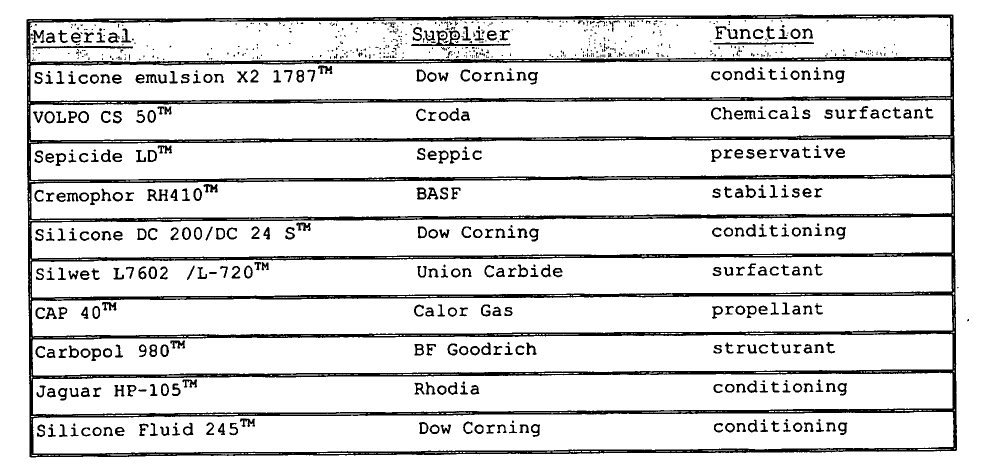

Ethanol used as SD Alcohol 40-B (92% active)

### Example 4

A styling mousse is formulated as follows:

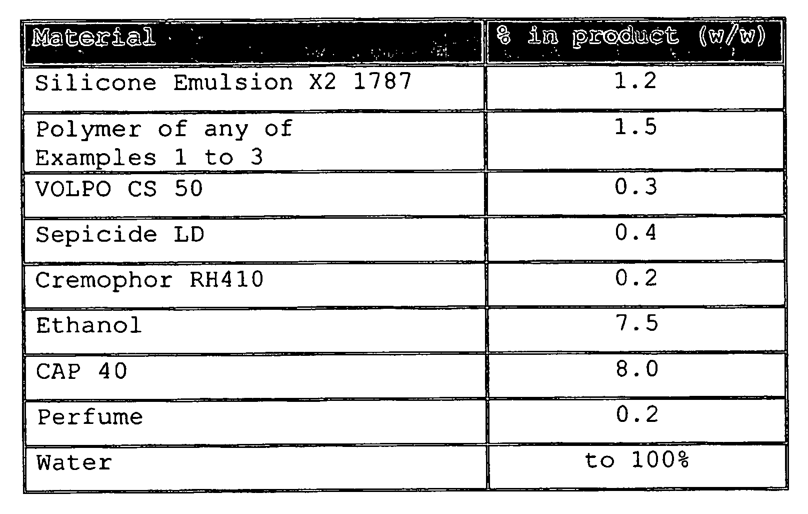

### Example 5

A hairspray is formulated as follows:

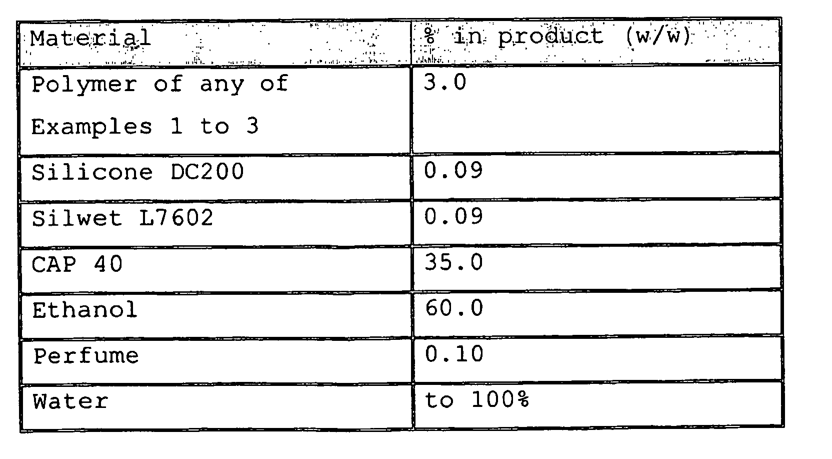

### Example 6

A pump spray is formulated as follows:

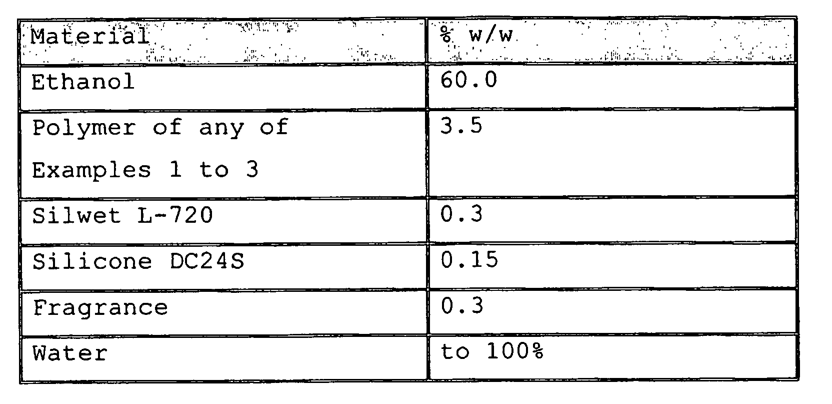

### Example 7

A styling gel is formulated as follows:

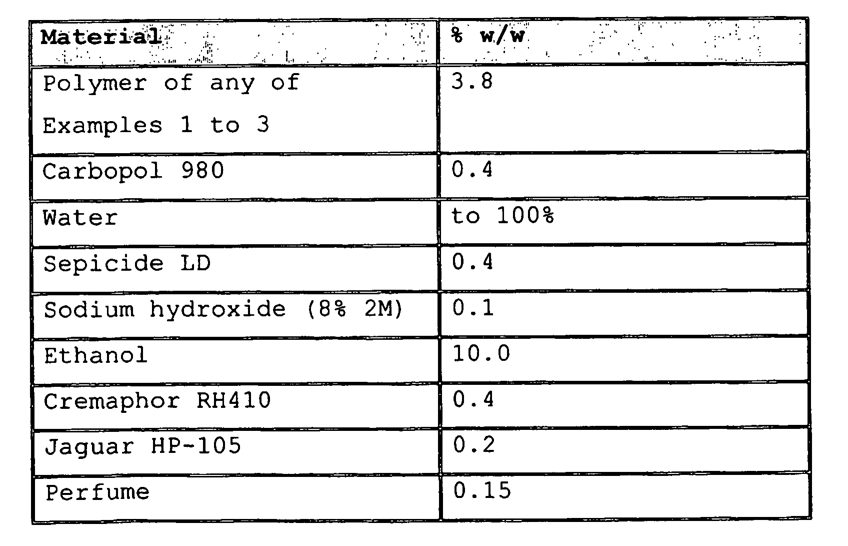

### Example 8

A 55% voc propelled aerosol composition is formulated as follows:

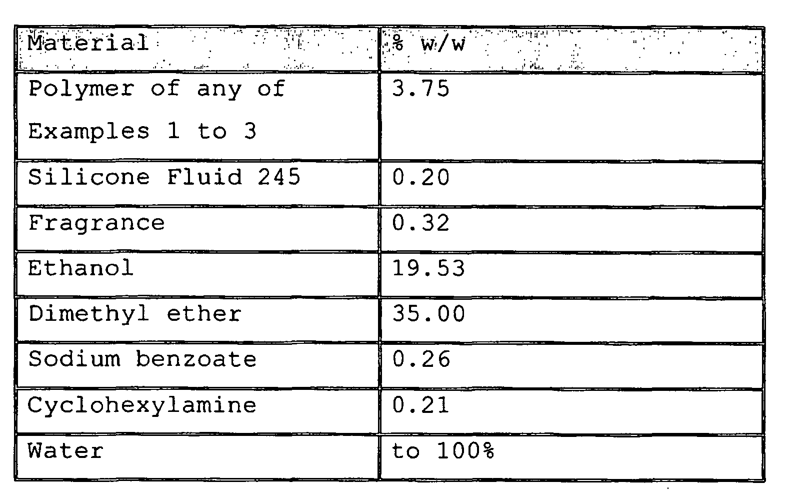

### Example 9

A 55% voc pump hairspray composition is formulated as follows:

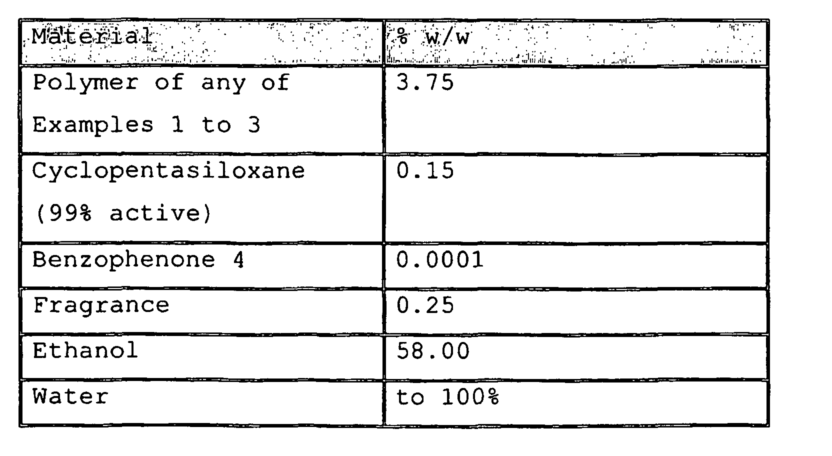

## Claims

1. A hair treatment composition comprising
i) a polymer comprising an ABA block Copolymer, wherein the A groups are polyacrylate blocks or polymethacrylate blocks and the B group is a poly(ethylene glycol).
ii) a cosmetically acceptable diluent or carrier.

2. A hair treatment composition as claimed in Claim 1, wherein the A groups of the polymer are poly(aminoalkyl methacrylate) blocks.

3. A hair treatment composition as claimed in Claim 2, wherein the A groups of the polymer are poly(2-(dimethylamino)ethyl methacrylate) blocks.

4. A hair treatment composition as claimed in any one of Claims 1 to 3, wherein each A block of the polymer is connected to the B block by a divalent linker group.

5. A hair treatment composition as claimed in Claim 4, wherein the divalent linker group is a carbonylalkylene group containing from 2 to 7 carbon atoms which forms an ester linkage to the B block.

6. A hair treatment composition as claimed in Claim 4, wherein the divalent linker group is a group of formula -C(O)-C(CH₃)₂-.

7. A hair treatment composition as claimed in any one of the preceding claims wherein the polymer is soluble in a solvent selected from water, ethanol and mixtures thereof.

8. A hair treatment composition as claimed in any preceding claim, further comprising a fragrance or perfume.

9. Composition as claimed in any preceding claim which is a hair styling composition.

10. Composition as claimed in any preceding claim which comprises from 0.1 to 10% by weight of the polymer.

11. Composition as claimed in any preceding claim further comprising an additional hair styling polymer.

12. Composition as claimed in any preceding claim, further comprising from 0.01% to 7.5% by weight of a surfactant.

13. Composition as claimed in any preceding claim, further comprising up to 30% by weight of a propellant.

14. Composition as claimed in any preceding claim which is a hair styling cream or gel including from 0.01% to 10% by weight of a structurant or thickener.

15. A cosmetic method of treating hair which comprises applying to the hair a composition according to any preceding claim.

16. Use of a composition according to any one of Claims 1 to 13 for the cosmetic treatment of hair.

## Patentansprüche

1. Haarbehandlungszusammensetzung, umfassend
(i) ein Polymer; das ein ABA-Blockcopolymer enthält, in dem die A-Gruppen Polyacrylatblöcke oder Polymethacrylatblöcke sind und die B-Gruppe ein Poly(ethylenglycol) ist.
(ii) einen kosmetisch verträglichen Verdünner oder Träger.

2. Haarbehandlungszusammensetzung nach Anspruch 1, in der die A-Gruppen des Polymers Poly(aminoalkylmethacrylat)blöcke sind.

3. Haarbehandlungszusammensetzung nach Anspruch 2, in der die A-Gruppen des Polymers Poly(2-(dimethylamino)ethylmethacrylat)blöcke sind.

4. Haarbehandlungszusammensetzung nach einem der Ansprüche 1 bis 3, in der jeder A-Block des Polymers durch eine zweiwertige Verknüpfungsgruppe mit dem B-Block verbunden ist.

5. Haarbehandlungszusammensetzung nach Anspruch 4, in der die zweiwertige Verknüpfungsgruppe eine Carbonylalkylengruppe mit 2 bis 7 Kohlenstoffatomen ist, die eine Esterbindung zum B-Block herstellt.

6. Haarbehandlungszusammensetzung nach Anspruch 4, in der die zweiwertige Verknüpfungsgruppe eine Gruppe der Formel -C(O)-C(CH₃)₂- ist.

7. Haarbehandlungszusammensetzung nach einem der vorstehenden Ansprüche, in der das Polymer in einem aus Wasser, Ethanol und Gemischen davon ausgewählten Lösungsmittel löslich ist.

8. Haarbehandlungszusammensetzung nach einem der vorstehenden Ansprüche, die außerdem einen Duftstoff oder ein Parfum umfasst.

9. Zusammensetzung nach einem der vorstehenden Ansprüche, bei der es sich um eine Haarstylingzusammensetzung handelt.

10. Zusammensetzung nach einem der vorstehenden Ansprüche, die 0,1 bis 10 Gew.-% des Polymers umfasst.

11. Zusammensetzung nach einem der vorstehenden Ansprüche, die außerdem ein zusätzliches Haarstylingpolymer umfasst.

12. Zusammensetzung nach einem der vorstehenden Ansprüche, die außerdem 0,01 bis 7,5 Gew.-% eines Tensids umfasst.

13. Zusammensetzung nach einem der vorstehenden Ansprüche, die außerdem bis zu 30 Gew.-% eines Treibmittels umfasst.

14. Zusammensetzung nach einem der vorstehenden Ansprüche, bei der es sich um eine Haarstylingcreme bzw. ein Haarstylinggel mit 0,01 bis 10 Gew.-% eines strukturbildenden Mittels oder Verdickungsmittels handelt.

15. Kosmetisches Verfahren zur Behandlung von Haar, das das Aufbringen einer Zusammensetzung nach einem der vorstehenden Ansprüche auf das Haar umfasst.

16. Verwendung einer Zusammensetzung nach einem der Ansprüche 1 bis 13 zur kosmetischen Behandlung von Haar.

## Revendications

1. Composition de traitement du cheveu, comprenant
i) un polymère comprenant un copolymère séquencé ABA, dans lequel les groupes A sont des motifs polyacrylate ou des motifs polyméthacrylate et le groupe B est un polyéthylène glycol.
ii) un diluant ou un véhicule cosmétiquement acceptable.

2. Composition de traitement du cheveu selon la revendication 1, dans laquelle les groupes A du polymère sont des motifs polyméthacrylate d'aminoalkyle.

3. Composition de traitement du cheveu selon la revendication 2, dans laquelle les groupes A du polymère sont des motifs polyméthacrylate de 2-(diméthylamino)- éthyle.

4. Composition de traitement du cheveu selon l'une quelconque des revendications 1 à 3, dans laquelle chaque motif A du polymère est relié au motif B par un groupe de liaison divalent.

5. Composition de traitement du cheveu selon la revendication 4, dans laquelle le groupe de liaison divalent est un groupe carbonylalkylène qui contient de 2 à 7 atomes de carbone et qui forme un pont ester vers le motif B.

6. Composition de traitement du cheveu selon la revendication 4, dans laquelle le groupe de liaison divalent est un groupe de la formule -C(O)-C(CH₃)₂-.

7. Composition de traitement du cheveu selon l'une quelconque des revendications précédentes, dans laquelle le polymère est soluble dans un solvant choisi parmi l'eau, l'éthanol et des mélanges de ceux-ci.

8. Composition de traitement du cheveu selon l'une quelconque des revendications précédentes, comprenant en plus une senteur ou un parfum.

9. Composition selon l'une quelconque des revendications précédentes, qui est une composition de coiffure.

10. Composition selon l'une quelconque des revendications précédentes, qui comprend de 0,1 à 10% en poids du polymère.

11. Composition selon l'une quelconque des revendications précédentes, comprenant en plus un polymère de coiffure supplémentaire.

12. Composition selon l'une quelconque des revendications précédentes, comprenant en plus de 0,01% à 7,5% en poids d'un agent tensio-actif.

13. Composition selon l'une quelconque des revendications précédentes, comprenant en plus jusqu'à 30% en poids d'un propulseur.

14. Composition selon l'une quelconque des revendications précédentes, qui est une crème ou un gel de coiffure comprenant de 0,01% à 10% en poids d'un agent structurant ou épaississant.

15. Procédé cosmétique de traitement du cheveu, qui comprend l'application au cheveu d'une composition selon l'une quelconque des revendications précédentes.

16. Utilisation d'une composition selon l'une quelconque des revendications 1 à 13, pour le traitement cosmétique du cheveu.
